Europäisches Patentamt

European Patent Office

Office européen des brevets

(11) Veröffentlichungsnummer : **0 417 722 B1**

(19)

(12) # EUROPÄISCHE PATENTSCHRIFT

(45) Veröffentlichungstag der Patentschrift :
**10.11.93 Patentblatt 93/45**

(51) Int. Cl.⁵ : **B01J 35/02**, B01J 27/192,
C07C 51/25, B01J 35/10

(21) Anmeldenummer : **90117464.9**

(22) Anmeldetag : **11.09.90**

(54) **Katalysator für die katalytische Gasphasenoxidation von Olefinen zu ungesättigten Aldehyden.**

(30) Priorität : **13.09.89 DE 3930533**

(43) Veröffentlichungstag der Anmeldung :
**20.03.91 Patentblatt 91/12**

(45) Bekanntmachung des Hinweises auf die
Patenterteilung :
**10.11.93 Patentblatt 93/45**

(84) Benannte Vertragsstaaten :
**AT BE CH DE DK ES FR GB GR IT LI LU NL SE**

(56) Entgegenhaltungen :
**EP-A- 0 102 641
EP-A- 0 239 071
EP-A- 0 279 374
EP-A- 0 296 285
EP-A- 0 355 664
US-A- 4 510 261**

(73) Patentinhaber : **Degussa Aktiengesellschaft
Weissfrauenstrasse 9
D-60311 Frankfurt (DE)**

(72) Erfinder : **Böck, Wolfgang, Dr.
Am Häuser Graben 2
D-6456 Langenselbold (DE)**
Erfinder : **Arntz, Dietrich, Dr.
Lorsbachstrasse 32
D-6370 Oberursel (DE)**
Erfinder : **Prescher, Günter, Dr.
10 Sherwood Drive
Larchmont, N.Y. 10538-2626 (US)**
Erfinder : **Burkhardt, Werner
Reichenbachstrasse 9
D-6486 Brachttal 2 (DE)**

## Beschreibung

Die Erfindung betrifft einen Katalysator für die heterogen katalysierte Gasphasenoxidation von Propen zu Acrolein, der in Rohrbündelreaktoren eingesetzt wird.

Die stark exotherme Umsetzung von Propen an heterogenen Katalysatoren mit einem Sauerstoff enthaltenden Gas führt neben dem erwünschten Produkt Acrolein zu einer Reihe von unerwünschten Nebenprodukten. Es ist bekannt, daß durch eine gute Abführung der Reaktionswärme, beispielsweise in Rohrbündelreaktoren, lokale Überhitzungen des Katalysators und damit einhergehende erhöhte Nebenproduktbildung vermieden werden kann.

Es ist weiterhin bekannt, daß durch Größe und äußere Form der Katalysatorkörper der Druckverlust einer Katalysatorschüttung beeinflußt werden kann. Die innere Struktur der Katalysatorkörper (Porosität, Länge der Diffusionswege) bestimmt entscheidend den Stoff- und Wärmetransport im Katalysator und hat damit neben der Zusammensetzung der katalytisch aktiven Masse maßgeblichen Einfluß auf die Selektivität. Hohe Druckfestigkeit und Abriebfestigkeit ist eine Vorbedingung, um einen Katalysator in der industriellen Praxis einsetzen zu können. Ein hoher Abrieb würde dazu führen, daß beim Füllen der Rohre eines Rohrbündelreaktors die Streuung der Druckverluste der Einzelrohre hoch ist, woraus eine unterschiedliche Durchströmung derselben mit Beeinträchtigung der Selektivität resultiert.

In der DE-PS 31 25 061 wird ein Verfahren zur Herstellung von Acrolein unter Einsatz von Schalenkatalysatoren beschrieben.

Bei Schalenkatalysatoren werden durch die temperaturausgleichende Wirkung des inerten Trägers lokale Überhitzungen vermieden; in der relativ dünnen Schale sind die Diffusionswege für die gasförmigen Reaktanden kurz.

In der DE-OS 33 38 380 werden für die Oxidation von Propen zu Acrolein ring- bzw. hohlzylinderförmige Katalysatoren beschrieben, die aus einer Mo, Fe, Bi und W enthaltenden Masse gefertigt werden. Diese Katalysatoren kann man sich aus den Schalenkatalysatoren abgeleitet vorstellen, indem der inerte Kern der Schalenkatalysatoren durch einen "inerten Hohlraum" ersetzt wird und die Schale an zwei gegenüberliegenden Stellen offen für den Zutritt der Reaktanden zum Hohlraum ist. Gegenüber den Schalenkatalysatoren weisen diese ring- bzw. hohlzylinderartigen Katalysatoren ein vergrößertes Verhältnis von äußerer Oberfläche zu Volumen auf. Die aktive Masse ist dadurch für die Reaktanden besser zugänglich. Der niedrige Druckverlust und die hohe Wärmeabfuhr der Schalenkatalysatoren liegen auch hier vor. Um bei den "Hohlkatalysatoren" eine genügende mechanische Festigkeit zu erzielen, wird die aktive Masse stark verdichtet, was dazu führt, daß die innere Struktur nachteilig beeinflußt wird.

Katalysatoren in Ringform, hier allerdings mit abgerundeten Stirnflächen zwecks Verbesserung der Verfüllbarkeit, werden auch in der EP-OS 0 184 790 beschrieben, wobei jedoch weder die Katalysatormasse noch ein spezielles Herstellverfahren angegeben sind, insbesondere sind keine Maßnahmen zur Erzielung einer speziellen günstigen inneren Struktur dargestellt.

Um die aktive Masse optimal nutzen zu können, muß die innere Struktur des Katalysators so aufgebaut sein, daß eine an sich mögliche hohe Reaktionsgeschwindigkeit nicht durch eine Behinderung des Stofftransports im Inneren des Katalysators begrenzt wird.

Ein in dieser Richtung gehender Versuch ist in der EP-OS 0 279 374 gezeigt. Hier ist ein Herstellungsverfahren für einen Mo, Fe, Bi enthaltenden, durch spezifische Oberfläche, Porenvolumen und Porenverteilung charakterisierten Katalysator, beschrieben. Verfahrensbedingt können jedoch nur Katalysatorpartikel von annähernd kugelförmiger Form, d. h. mit sehr kleinem Verhältnis von Oberfläche zu Volumen erhalten werden, oder die Partikel müßten sehr klein werden. Dem sind aber für die industrielle Anwendung wegen des damit verbundenen hohen Druckverlustes Grenzen gesetzt.

Die nach bekanntem Stand der Technik hergestellten und verwendeten Katalysatoren haben bezüglich der dargelegten Gesichtspunkte einige Nachteile. Durch Einsatz verschieden geformter Körper wird entweder versucht, die Diffusionswege zu verkürzen, lokale Überhitzungen zu vermeiden oder durch eine geeignete innere Struktur des Katalysators eine verbesserte Ausnutzung der Katalysatorvolumina zu erreichen.

Einzelmaßnahmen dieser Art haben bisher dazu geführt, daß sich mit solchen Katalysatoren im technischen Einsatz nur eine vergleichsweise unbefriedigende Produktivität bei der Acroleingewinnung pro eingesetztem Katalysatorvolumen erreichen läßt. Dies ist wirtschaftlich von erheblichem Nachteil, da zum Ausgleich große und kostspielige Reaktoren mit hohem Füllvolumen für die Katalysatoren zur Durchführung der Reaktion eingesetzt werden müssen.

Der Erfindung liegt die Aufgabe zugrunde, einen Katalysator zur Herstellung von ungesättigten Aldehyden aus Olefinen, insbesondere von Acrolein aus Propen durch Oxidation mit einem molekularen Sauerstoff enthaltenden Gas zu schaffen, welcher mit einem großen Verhältnis von geometrischer Oberfläche zu Volumen und gleichzeitig geringem niedrigen Diffusionswiderstand diese Nachteile überwindet und insbesondere eine überraschend hohe Produktivität ermöglicht.

Der erfindungsgemäße Katalysator ist durch die Kombination folgender Eigenschaften gekennzeichnet:

a) eine zumindest die Elemente Molybdän, Wismut, Phosphor und Sauerstoff in Atomverhältnissen

$$Mo_{12} Bi_{0,2-10} P_{0,2-5}O_x$$

sowie siliciumhaltiges Trägermaterial enthaltende aktive Masse;

b) einen Katalysator von beliebiger geometrischer Form, dessen Verhältnis von äußerer Oberfläche $O_p$ zu Volumen $V_p$ oberhalb 1,6 mm$^{-1}$ liegt und dessen räumliche Ausdehnung, beschrieben durch den Durchmesser einer ihn gerade noch umschließenden Kugel, kleiner als 7,5 mm ist;

c) eine Porosität des Katalysatorkörpers von mindestens 0,46,

die Abwesenheit von Mikroporen (< 2 nm),

ein Mesoporenvolumen (2 - 30 nm) von mindestens 0,03 cm$^3$/g, vorzugsweise 0,03 - 0,10 cm$^3$/g,

sowie ein Makroporenvolumen (> 30 nm) von mindestens 0,30 cm$^3$/g, vorzugsweise 0,30 - 0,50 cm$^3$/g;

d) eine Quecksilberdichte des Katalysatorkörpers von mindestens 1,25 g/cm$^3$, vorzugsweise 1,25 - 1,85 g/cm$^3$;

e) eine spezifische Oberfläche nach BET von mindestens 10 m$^2$/g, vorzugsweise 10 - 25 m$^2$/g;

f) eine Bruchfestigkeit von mindestens 6.0 N;

g) einen Abrieb von unter 50 mg/g Katalysator;

h) einen Druckverlust von unter 1600 Pa/m einer in einem Rohr mit 2 cm Durchmesser eingebrachten Katalysatorschüttung.

Der erfindungsgemäße Katalysator weist in allen für die relevante Gasphasenoxidation geeigneten Mischoxidformulierungen, welche zumindest Molybdän, Wismut und Phosphor in den beanspruchten Atomverhältnissen enthalten, große Vorteile hinsichtlich der erzielbaren Raumzeitausbeute auf. Die für eine hohe Raumzeitausbeute erforderliche Aktivität wird durch das günstige Verhältnis zwischen äußerer Oberfläche und Volumen und die günstige innere Struktur ermöglicht. Durch das vergrößerte Verhältnis von Oberfläche zu Volumen ist die jeweilige katalytisch aktive Masse für die Reaktanden gut zugänglich und die Diffusionswege sind kurz. Die günstige innere Struktur hat zur Folge, daß der Diffusionswiderstand im Katalysatorinneren gering ist.

Die durch die große innere Oberfläche bedingte hohe Aktivität kann dadurch gut ausgenutzt werden. Grundsätzlich hat der herabgesetzte Diffusionswiderstand im Katalysatorkörper auch einen günstigen Einfluß auf die Selektivität.

Der Körper des erfindungsgemäßen Katalysators kann beliebige geometrische Formen haben. Beispiel für bevorzugte Körperformen sind in der anliegenden Zeichnung gezeigt.

Von den inzwischen entwickelten Katalysatorformulierungen mit den erwähnten Grundbestandteilen hat sich eine Masse, enthaltend die Zusammensetzung

$$Mo_{12}Fe_{0,4-4,0}Co_{0,4-4,0}Ni_{1,0-9,0}Bi_{0,2-2,0}$$
$$P(As)_{0,2-2,0}K(Rb,Cs)_{0-0,1} Sm_{0,01-0,2}Si_{5-40}O_x,$$

wobei das Element Silicium als pyrogene oder hochdisperse gefällte Kieselsäure, Kieselsäuresol, feinteiliges Aluminiumsilikat, insbesondere in Form von Montmorillonit vorliegt, als besonders geeignet erwiesen.

Eine bevorzugte Variante dieser Formulierung besteht aus einer Masse, enthaltend die Zusammensetzung

$$Mo_{12}Fe_{0,6-2,0}Co_{0,6-2,0}Ni_{2,0-6,0}Bi_{0,5-1,5}$$
$$P(As)_{0,5-1,5}K(Rb,Cs)_{0,001-0,05}Sm_{0,02-0,1}Si_{10-30}O_x,$$

wobei das Element Silicium in Form von pyrogenem SiO$_2$ und Montmorillonit im Gewichtsverhältnis 1 : 0,5 bis 1 : 4 vorliegt.

Dabei hat es sich als zweckmäßig erwiesen, daß der Montmorillonit eine durch Glühbehandlung herabgesetzte spezifische Oberfläche nach BET unterhalb von 2,0 m$^2$/g hat.

Ein weiterer Gegenstand der Erfindung ist ein Herstellungsverfahren für den beschriebenen Katalysator in seiner Grundzusammensetzung und den weiter entwickelten Varianten.

Es ist dadurch gekennzeichnet, daß man

a) eine in an sich bekannter Weise durch Vereinigen von Salzlösungen der katalytisch aktiven Elemente (außer Si) gewonnenen Suspension des Kopräzipitats mit dem unlöslichen siliciumhaltigen Feststoff mischt und die dabei erhaltene Suspension unter Bedingungen sprühtrocknet, welche eine Anfangstemperatur der Trocknungsluft von 300° C - 600° C und eine Temperatur bei der Abscheidung des getrockneten Pulvers von 120° C - 220° C sowie eine ein Sprühkorn mit einem mittleren Korndurchmesser kleiner 30 µm erzeugende Zerstäubungsintensität vorsehen, wobei die Verweilzeit des Sprühkorns im Trockner 2 bis 25 sec. beträgt,

b) das trockene Sprühkorn in einem Ofen, vorzugsweise einem Drehrohrofen, bei Verweilzeiten von 5 bis 60 min und einer Spitzentemperatur im Sprühkorn von 320 bis 480°C kalziniert.

c) das kalzinierte Sprühkorn mit 5 bis 40 Gew.-%, bezogen auf die Menge des Sprühkorns, eines bei einer Temperatur unter 400°C vollständig zersetzlichen Porenbildners und mit einer eine extrusionsfähige Masse ergebenden Menge an Befeuchtungs-, Gleit- und Bindemittel, deren Anteil insgesamt nicht mehr als 40 Gew.-% der Sprühkornmenge ausmacht, bei einer Temperatur unterhalb 80° C in der gewünschten geometrischen Form bei einem Druck unterhalb 50 bar extrudiert und den extrudierten Strang durch Schneiden auf die Länge des gewünschten Körpers unterteilt,

d) die Einzelkörper trocknet, dann in einem Ofen, vorzugsweise in einem Drehrohrofen, die enthaltene zersetzbare Substanz vorsichtig ausbrennt und dann bei einer Verweilzeit von 5 bis 60 min und einer in der Schüttung der Einzelkörper gemessenen Spitzentemperatur von 450 bis 650°C im Luftstrom tempert.

Das Verfahren erschließt eine Vorgehensweise, die im wesentlichen aus der Kombination einer Sprühtrocknung des Ausgangsmaterials bei definierten Bedingungen, einer Zwischentemperung innerhalb einer angegebenen Temperaturspanne, einer Extrusion des kalzinierten Sprühkorns mit definierten Mengen eines Porenbildners und üblichen Verarbeitungshilfsmitteln in gewichtsmäßig begrenzter Menge unter genauen Extrusionsbedingungen und einer abschließenden Temperung an der Luft bei angehobenem Temperaturniveau besteht. Nach einer günstigen Ausführungsform des erfindungsgemäßen Verfahrens wird als Porenbildner fester Pentaerythrit mit einem mittleren Korndurchmesser kleiner 40 µm eingesetzt. Neben Pentaerythrit können als Porenbildner auch Cellulosepulver, Harnstoff, Oxalsäure und Polyvinylalkohol eingesetzt werden.

Durch die Sprühtrocknung der Suspension des Kopräzipitats wird bei den angegebenen Bedingungen ein kugeliges Sprühkorn mit hoher innerer Porosität erhalten. Dadurch wird die für solche Katalysatoren hohe innere Oberfläche erzeugt. Durch die Zwischentemperung werden sämtliche zersetzlichen Bestandteile aus dem Primärkorn ausgetrieben, damit bei der abschließenden Temperung keine Verringerung der Festigkeit auftreten kann.

Durch den beim Extrusionsprozeß, vorzugsweise im Korngrößenbereich des Primärkorns zugefügten Porenbildner, entsteht bei der Endtemperung ein Makroporensystem, durch das die hochaktiven mesoporösen Primärpartikel für die Reaktanden gut zugänglich werden. Beim Extrudieren können mit Vorteil als Gleitmittel Petroleum oder Wasser und als Befeuchtungs - und Bindemittel bzw. Gleitmittel eine 1 - 10%ige wäßrige Methylcelluloselösung, vorzugsweise in Form einer Öl-in-Wasser-Emulsion oder als Bindemittel trockenes Methylcellulosepulver eingesetzt werden.

Die abschließende Temperung bei 450 - 650° C schließt einen vorausgehenden vorsichtigen Ausbrennvorgang während des Aufheizens ein. Das Aus brennen und Tempern der extrudierten Einzelkörper kann aber auch in gesonderten Schritten vorgenommen werden. In beiden Fällen werden besonders gute Ergebnisse erhalten, wenn man die extrudierten Einzelkörper und den Luftstrom beim Ausbrennvorgang im Gleichstrom führt und das Ausbrennen bei höchstens 400° C vornimmt.

Bei Einsatz der neuen Katalysatoren für die Oxidation von Olefinen zu ungesättigten Aldehyden mittels eines molekularen Sauerstoff enthaltenden Gases, z. B. bei der hier bevorzugten Darstellung von Acrolein aus Propen, können sehr günstige Betriebsbedingungen, welche zu einer hohen Produktivität führen, eingesetzt werden. Die Reaktion wird bei einer Temperatur von 300 - 380 C und einem Druck von 1 ,4 - 2,2 bar durchgeführt. Dabei werden die Reaktionspartner Propen, Luft, Inertgas, Wasser im Verhältnis 1 : 6-9 3-12 : 0-5 bei einer spezifischen Belastung von 2 - 8 mol Propen/dm³ Katalysatorschüttung/h der Katalysatorschüttung zugeführt. Anstelle von Inertgas wird vorzugsweise das Abgas aus der Reaktion verwendet, aus dem die kondensierbaren Bestandteile abgetrennt wurden. Besonders gute Ergebnisse werden bei Einsatz von Rohrbündelreaktoren erhalten, deren Rohre einen Innendurchmesser von 16 - 25 mm aufweisen.

Die Erfindung wird nachfolgend anhand von Ausführungsbeispielen erläutert:

Die in den Beispielen zitierten Meßgrößen werden wie folgt bestimmt:

1) Bestimmung der Porosität:

Sie wird aus Quecksilber- und Helium-Dichte berechnet;

Porosität = (1 - Hg-Dichte/He-Dichte) · 100 (dimensionslos)

"Porosität" ist als der prozentuale Anteil des Leervolumens der Katalysatormasse an deren Gesamtvolumen definiert.

2) Bestimmung des Mesoporenvolumens:

Barrett, E. P. ; Joyner, L.G. ; Halenda, P. P.

J. Am. Chem. Soc. , 73 (1951 ) , S. 373;

3) Bestimmung des Makroporenvolumens:

Hg-Einpreßverfahren mit Carlo-Erba Porosimeter 200 bis 1000 bar Druck;

4) Bestimmung der Schüttdichte, Quecksilberdichte (Scheindichte) und Heliumdichte (wahre Dichte);

Die Schüttdichte wird bestimmt, indem ein gerades Stahlrohr von 20 mm Innendurchmesser innerhalb 1 min. gleichmäßig mit 200 g Katalysator befüllt wird und die Höhe der resultierenden Katalysatorschüttung gemessen wird.

Die Quecksilberdichte wird bestimmt, indem in ein Pyknometer von 25 ml Volumen 2 g des auf 200 $\mu$m zerkleinerten Katalysators eingefüllt werden und dann das Pyknometer mit Quecksilber sorgfältig gefüllt wird. Aus den Massen des Quecksilbers, die zur Füllung des Pyknometers mit bzw. ohne Katalysastorprobe benötigt werden und der Masse der Katalysatorprobe selbst, wird die Quecksilberdichte (oder Scheindichte) des Katalysators erhalten. Die Heliumdichte (oder wahre Dichte) der Katalysatormasse wird mit einem Beckmann-Luftvergleichspyknometer bestimmt.

5) Bestimmung der BET-Oberfläche nach DIN 66131 (Meßgas $N_2$):

Ausheizbedingungen:     15 h bei 100° C getrocknet,

1 h bei 200°C im Vakuum entgast;

6) Bestimmung der Bruchfestigkeit:

Messung senkrecht zur Extrusionsrichtung mit Erweka TBA 28, arithmetisches Mittel aus 100 Einzelmessungen ± Standardabweichung;

7) Bestimmung des Abriebs:

Messung mit Roche Friabilator TA3-R, Einwaage 50 g, 10 Upm, Belastungsdauer 5 min; Ergebnisangabe als Abrieb von Katalysatorteilchen < 1 mm in mg/g Katalysator;

8) Bestimmung des Druckverlusts:

In ein Rohr von 2 cm Innendurchmesser, welches am unteren Ende mit einem Drahtnetz verschlossen ist, wird gleichmäßig innerhalb einer Minute so viel Katalysator eingefüllt, daß die Höhe der Katalysatorschüttung 1 m beträgt. Diese Schüttung wird mit 1 Nm³/h Luft von 20° C durchströmt und der Druckverlust gemessen;

9) Bestimmung der Korngrößenverteilung:

Sie erfolgt mit einem CILAS Granulometer 715. Als Suspensionsflüssigkeit wird Äthanol eingesetzt. Zur Zerstörung von Agglomeraten wird 1 min. mit Ultraschall desagglomeriert.

10) Bestimmung der katalytischen Eigenschaften:

Die katalytische Wirkung der fertigen Katalysatoren wird in einem technischen Reaktorrohr von 20,5 mm Innendurchmesser, das von außen durch ein Salzbad gekühlt ist, bei einer Katalysatorschüttungslänge von 250 cm anhand der Umsetzung von Propen zu Acrolein getestet. Die Reaktion wird bei einer Einspeisung von 5,8 mol Propen/h (bzw. 5,2 mol Propen/h, gekennzeichnet mit * in Tabelle 3) 43,5 mol Luft/h, 34,8 mol Abgas (Zusammensetzung: 5 % $O_2$, 1 % Propen, 94 % Inertgas ($CO_2$, CO, $N_2$, Ar, Propan)) und 2,9 mol $H_2O$/h und einem Druck von 1 ,8 bar am Eintritt in die Katalysatorschüttung durchgeführt. Die eingestellte Salzbadtemperatur, die maximal erhaltene Übertemperatur (Exotherme) in der Rohrmitte und die gemessenen Umsätze und Ausbeuten sind in Tab. 3 zusammengestellt.

Dabei ist definiert

die Acroleinausbeute (%) als

$$\frac{\text{mol/h Acrolein gebildet}}{\text{mol/h Propen zugeführt}} \cdot 100$$

die Acrylsäureausbeute (%) als

$$\frac{\text{mol/h Acrylsaüre gebildet}}{\text{mol/h Propen zugeführt}} \cdot 100$$

der Umsatz von Propen % als

$$1 - \frac{\text{mol/h aus dem Reaktionsrohr austretendes Propen}}{\text{mol/h in das Reaktionsrohr eingespeistes Propen}} \cdot 100$$

die Acroleinselektivität (%) als

$$\frac{\text{Acroleinausbeute}}{\text{Propenumsatz}} \cdot 100$$

die Acrylsäureselektivität (%) als

$$\frac{\text{Acrylsaüreausbeute}}{\text{Propenumsazt}} \cdot 100$$

und

die Acroleinproduktivität als

$$\frac{\text{g/h Acrolein gebildet}}{\text{dm}^3 \text{ Katalysatorschüttung}}$$

Die folgenden Beispiele betreffen die Herstellung und Anwendung der in Tabelle 1 aufgeführten Katalysatoren.

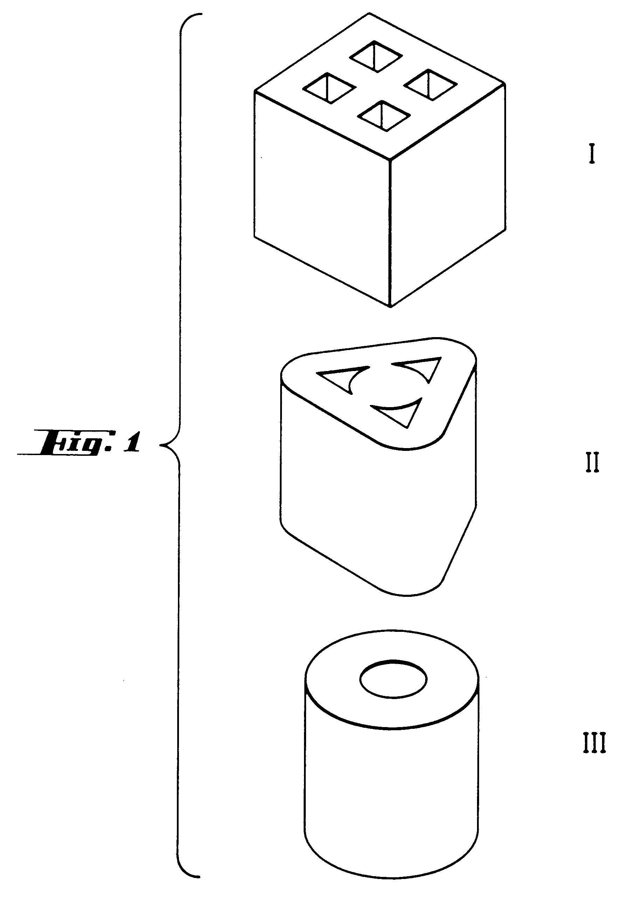

Die in Tabelle 1 unter der Beispiel-Nr. 1 angegebene Katalysatorzusammensetzung, die bereits in DE-PS 11 29 150 beschrieben ist, wurde in der geometrischen Form III von Fig. 1 hergestellt.

Beispiel 1

Das Kopräzipitat für die Herstellung der aktiven Katalysatorphase wird erhalten, indem man 4918 g Bi(NO$_3$)$_3$ · 5H$_2$O, 2299 g H$_2$MoO$_4$ und 134 g 85 % H$_3$PO$_4$ in 3380 g Wasser, das zuvor mit 460 g konz. HNO$_3$ versetzt wurde, bei Raumtemperatur und kräftigem Rühren einträgt. Die entstehende Suspension wird in 6338 g wäßrige 30 %ige Kieselsolsuspension gegeben. Die Suspension wird mit 4250 g Wasser verdünnt und in einem Sprühtrockner bei einer Eintrittstemperatur der Trocknungsluft von 500° C getrocknet. Das Verhältnis von Trocknungsluftmenge zu versprühter Suspensionsmenge wird so eingestellt, daß eine Verweilzeit von 6 sec. des Sprühkorns im Sprühtrockner erhalten wird. Die Zerstäubungsintensität ist so gewählt, daß ein Sprühkorn mit einem mittleren Korndurchmesser $\overline{dp}$ = 6 μm erhalten wird.

Das getrocknete Sprühkorn wird in einem Drehrohrofen bei einer Verweilzeit von 60 min. bei einer maximalen Temperatur im Sprühkorn von 350° C kalziniert.

1,5 kg des kalzinierten Pulvers werden in einer Kneter/Extruderkombination vorgelegt und mit 0,375 kg Pentaerythrit ("feinstgemahlen") mit einem mittleren Korndurchmesser von $\overline{dp}$ = 40 μm 5 min. gemischt. Zu dieser Mischung werden 600 g einer 10 %igen Tyloselösung gegeben, in der zuvor 100 g Petroleum emulgiert wurden. Diese Masse wird nun solange geknetet, bis ein homogener plastischer Zustand erreicht ist. Die Extrusion erfolgt dann bei einem Druck von 10 bar und einer Temperatur von 20° C in der extrudierten Masse. Der extrudierte Hohlstrang wird nach jeweils 5 mm abgeschnitten, die abgeschnittenen Ringe von 5 mm Durchmesser, 5 mm Länge und 1 , 8 mm Innendurchmesser bei Raumtemperatur getrocknet.

Die getrockneten Ringe werden in ein beheiztes Drehrohr eingetragen und bei einer Drehzahl von 2 min$^{-1}$ und Luftüberschuß auf eine Spitzentemperatur von 550° C in der durch das Drehrohr wandernden Schüttung erhitzt. Die Verweilzeit im Drehrohr beträgt dabei 60 min.

Die an der Katalysatorzusammensetzung bzw. deren Vorstufen gemessenen physikalischen Eigenschaften sind in Tabelle 2 und die Katalysatorwirkung bei der Herstellung von Acrolein in Tabelle 3 zusammengefaßt.

Im folgenden Vergleichsbeispiel 1 wird beschrieben, wie die Katalysatorzusammensetzung der DE-PS 11 29 150 auf übliche Weise zu Tabletten von 5 x 5 mm verformt wird.

Vergleichsbeispiel 1

Das Kopräzipitat für die Herstellung der aktiven Katalysatorphase wird wie in Beispiel 1 hergestellt. Die anfallende Suspension wird im Rotationsverdampfer im Vakuum zur Trockne eingedampft. Der trockene Rückstand wird zerkleinert und durch ein 1 mm-Sieb abgesiebt. Dem erhaltenen Pulver werden 5 Gew.-% Stearinsäure als Preßhilfsmittel untergemischt. Die Mischung wird auf einer Tablettiermaschine zu Tabletten von 5 x 5 mm verformt. Die Tabletten werden in einem Umlufttrockenschrank unter zusätzlicher Frischluftzufuhr 16 h bei 538° C getempert.

Die in Tabelle 1 unter Beispiel-Nr. 2 angegebene Katalysatorzusammensetzung wurde in der geometrischen Form III von Fig. 1 wie folgt hergestellt:

Beispiel 2

Das Kopräzipitat für die Herstellung der aktiven Katalysatorphase wird erhalten, indem man 484,8 g Fe(NO$_3$)$_3$ · 9H$_2$O, 291 ,0 g Co(NO$_3$)$_2$ · 6H$_2$O, 1163,2 g Ni(NO$_3$)$_2$ · 6H$_2$O und 2,5 g KNO$_3$ in 3, 1 l Wasser löst und unter Rühren bei 90° C zunächst eine Lösung von 17,4 g SM$_2$O$_3$ in 106 g konz. HNO$_3$ zugibt. Zu dieser Lösung werden unter weiterem Rühren 601 g hochdisperse Kieselsäure (Aerosil 200) und 1202 g getemperter Montmorillonit (spez. Oberfläche nach BET < 1 m$^2$/g) gegeben. In einem getrennten Gefäß wird bei 60° C eine Lösung von 2118,6 g (NH$_4$)$_6$Mo$_7$O$_{24}$ · 6H$_2$O in 2,7 l H$_2$O bereitet und zu dieser unter intensivem Rühren 92,2 g 85 %ige H$_3$PO$_4$ gegeben.

Danach werden die beiden Lösungen unter intensivem Rühren vereinigt und eine Lösung von 242 ,5 g Bi(NO$_3$)$_3$ · 5H$_2$O in 204 g 8,2 %iger HNO$_3$ zugegeben. Die erhaltene Suspension wird in einem Sprühtrockner bei einer Eintrittstemperatur der Trocknungsluft von 550° C getrocknet. Das Verhältnis von Trocknungsluftmenge zu versprühter Suspensionsmenge wird dabei so eingestellt, daß eine Austrittstemperatur von 170° C erhalten wird und die Suspensionsmenge wird so eingestellt, daß eine Verweilzeit von 6 sec des Sprühkorns im Sprühtrockner erhalten wird. Die Zerstäubungsintensität ist so gewählt, daß ein Sprühkorn mit einem mittleren Durchmesser $\overline{dp}$ = 25 μm erhalten wird. Das getrocknete Sprühkorn wird in einem Drehrohrofen bei einer Verweilzeit von 30 min. und einer maximalen Temperatur im Sprühkorn von 420° C kalziniert.

1,6 kg des kalzinierten Sprühkorns werden in einer Kneter/Extruder-Kombination vorgelegt und mit 0,4 kg Pentaerythrit ("feinstgemahlen") eines mittleren Korndurchmessers von $\overline{dp}$ = 40 μm 5 min. gemischt. In dieser Mischung werden 493 g einer 6 Gew.-%igen Tyloselösung gegeben, in der zuvor 26,6 g Petroleum emulgiert wurden. Diese Masse wird nun solange geknetet, bis ein homogener plastischer Zustand erreicht ist. Die Extrusion erfolgt dann bei einem Druck von 10 bar und einer Temperatur von 30° C in der extrudierten Masse. Der extrudierte Hohlstrang wird nach jeweils 5 mm abgeschnitten, die abgeschnittenen Ringe von 5 mm Durchmesser, 5 mm Länge und 1,8 mm Innendurchmesser vorsichtig bei 80° C getrocknet.

Die getrockneten Ringe werden in ein beheiztes Drehrohr eingetragen und bei einer Drehzahl von 2 min$^{-1}$ und Luftüberschuß auf eine Spitzentemperatur von 600° C in der durch das Drehrohr wandernden Schüttung erhitzt. Die Verweilzeit im Drehrohr beträgt dabei 30 min.

Die an der Katalysatorzusammensetzung bzw. deren Vorstufen gemessenen physikalischen Eigenschaften sind in Tabelle 2 und die Katalysatorwirkung bei der Herstellung von Acrolein in der Tabelle 3 zusammengefaßt.

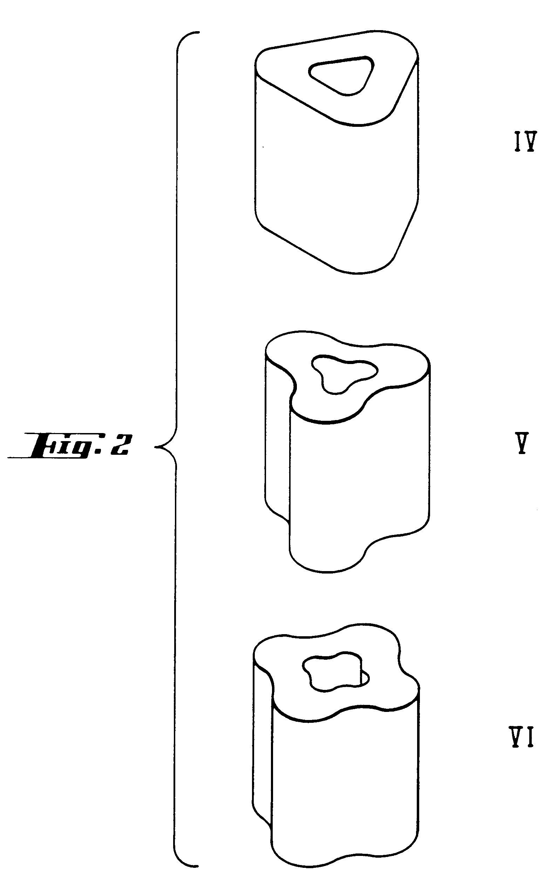

In Beispiel 3 wurde die gleiche Katalysatorzusammensetzung wie in Beispiel 2 in der geometrischen Form VI von Fig. 2 hergestellt.

Beispiel 3

Entsprechend Beispiel 2 wird das Kopräzipitat der aktiven Katalysatorphase hergestellt, getrocknet, kalziniert und in einer Kneter/Extruder-Kombination solange geknetet, bis ein homogener plastischer Zustand erreicht ist. Die Extrusion erfolgt bei einem Druck von 15 bar und einer Temperatur von 32° C in der extrudierten Masse. Der in Form VI von Fig. 2 extrudierte Hohlstrang wird nach jeweils 5 mm abgeschnitten, die abgeschnittenen Katalysatorrohlinge getrocknet und wie in Beispiel 2 im Drehrohr getempert.

In Beispiel 4 wurde die gleiche Katalysatorzusammensetzung wie in Beispiel 2 in der geometrischen Form II von Fig. 1 hergestellt.

Beispiel 4

Entsprechend Beispiel 2 wird das Kopräzipitat der aktiven Katalysatorphase hergestellt, getrocknet, kalziniert und in einer Kneter/Extruder-Kombination solange geknetet, bis ein homogener plastischer Zustand erreicht ist. Die Extrusion erfolgt bei einem Druck von 15 bar und einer Temperatur von 32° C in der extrudierten Masse. Der in Form II von Fig. 1 extrudierte Hohlstrang wird nach jeweils 5 mm abgeschnitten, die abgeschnittenen Katalysatorrohlinge getrocknet und wie in Beispiel 2 im Drehrohr getempert.

Im Vergleichsbeispiel 2 wurde die gleiche Katalysatorzusammensetzung wie in Beispiel 2 in Form von Stranspreßlingen extrudiert.

Vergleichsbeispiel 2

Entsprechend Beispiel 2 wird das Kopräzipitat der aktiven Katalysatorphase hergestellt, getrocknet, kalziniert und in einer Kneter/Extruder-Kombination solange geknetet, bis ein homogener plastischer Zustand erreicht ist. Die Extrusion erfolgt bei einem Druck von 8 bar und einer Temperatur von 25° C in der extrudierten Masse. Die in Form eines Vollstranges von 5 mm Durchmesser extrudierte Katalysatormasse wird nach jeweils 5 mm abgeschnitten, die abgeschnittenen Katalysatorrohlinge getrocknet und wie in Beispiel 2 im Drehrohr getempert.

In den folgenden Beispielen 5, 6 und 7 werden verschiedene Katalysatorzusammensetzungen in der geometrischen Form III von Fig. 1 wie in Beispiel 2 hergestellt.

Beispiel 5

Das Kopräzipitat für die Herstellung der aktiven Katalysatorphase wird erhalten, indem man 242,4 g Fe(NO$_3$ · 9H$_2$O, 232,8 g Co(NO$_3$)$_2$ · 6H$_2$O, 1744,8 g Ni(NO$_3$)$_2$ · 6H$_2$O und 5,1 g KNO$_3$ in 3,1 l Wasser löst und unter Rühren bei 90° C zunächst eine Lösung von 34,9 g Sm$_2$O$_3$ in 212 g konz. HNO$_3$ zugibt. In dieser Lösung werden unter weiterem Rühren 300,5 g hochdisperse Kieselsäure (Aerosil 200) und 300,5 g getemperter Montmorillonit (spez. Oberfläche nach BET < 1 m$^2$/g) gegeben. In einem getrennten Gefäß wird bei 60° C eine Lösung von 2118,6 g (NH$_4$)$_6$Mo$_7$O$_{24}$ · 6H$_2$O in 2,7 l H$_2$O bereitet und zu dieser unter intensivem Rühren 115,3 g 85 %ige H$_3$PO$_4$ gegeben. Danach werden die beiden Lösungen unter intensivem Rühren vereinigt und eine Lösung von 485. 1 g Bi(NO$_3$)$_3$ · 5H$_2$O in 408 g 8,2 %iger HNO$_3$ zugegeben. Die erhaltene Suspension wird in einem Sprühtrockner bei einer Eintrittstemperatur der Trocknungsluft von 550° C getrocknet. Das Verhältnis von Trocknungsluftmenge zu versprühter Suspensionsmenge wird dabei so eingestellt, daß eine Austrittstemperatur von 170° C erhalten wird und die Suspensionsmenge wird so eingestellt, daß eine Verweilzeit von 6 sec. des Sprühkorns im Sprühtrockner erhalten wird. Die Zerstäubungsintensität ist so gewählt, daß ein Sprühkorn mit einem mittleren Durchmesser $\overline{d}p$ = 25 μm erhalten wird. Das getrocknete Sprühkorn wird in einem Drehrohrofen bei einer Verweilzeit von 30 min. und einer maximalen Temperatur im Sprühkorn von 420° C kalziniert.

Die Weiterverarbeitung des kalzinierten Sprühkorns erfolgt wie in Beispiel 2 außer daß der extrudierte Hohlstrang einen Innendurchmesser von 2 mm hat.

### Beispiel 6

Das Kopräzipitat für die Herstellung der aktiven Katalysatorphase wird erhalten, indem man 808,0 g $Fe(NO_3)_3 \cdot 9H_2O$, 320, 1 g $Co(NO_3)_2 \cdot 6H_2O$, 1744,8 g $Ni(NO_3)_2$ $6H_2O$ und 0, 1 g $KNO_3$ in 3, 1 l Wasser löst und unter Rühren bei 90° C zunächst eine Lösung von 34,9 g $Sm_2O_3$ in 212 g konz. $HNO_3$ zugibt. In dieser Lösung werden unter weiterem Rühren 600,9 g hochdisperse Kieselsäure (Aerosil 200) und 1201,8 g getemperter Montmorillonit (spez. Oberfläche nach BET < 1 m²/g) gegeben. In einem getrennten Gefäß wird bei 60° C eine Lösung von 2118,6 g $(NH_4)_6Mo_7O_{24} \cdot 6H_2O$ in 2,7 l $H_2O$ bereitet und zu dieser unter intensivem Rühren 115,3 g 85 %ige $H_3PO_4$ gegeben. Danach werden die beiden Lösungen unter intensivem Rühren vereinigt und eine Lösung von 485, 1 g $Bi(NO_3)_3 \cdot 5H_2O$ in 408 g 8,2 %iger $HNO_3$ zugegeben. Die erhaltene Suspension wird in einem Sprühtrockner bei einer Eintrittstemperatur der Trocknungsluft von 550° C getrocknet. Das Verhältnis von Trocknungsluftmenge zu versprühter Suspensionsmenge wird dabei so eingestellt, daß eine Austrittstemperatur von 170° C erhalten wird und die Suspensionsmenge wird so eingestellt, daß eine Verweilzeit von 6 sec. des Sprühkorns im Sprühtrockner erhalten wird. Die Zerstäubungsintensität ist so gewählt, daß ein Sprühkorn mit einem mittleren Durchmesser $\overline{d}p$ = 25 μm erhalten wird. Das getrocknete Sprühkorn wird in einem Drehrohrofen bei einer Verweilzeit von 30 min. und einer maximalen Temperatur im Sprühkorn von 420° C kalziniert.

Die Weiterverarbeitung des kalzinierten Sprühkorns erfolgt wie in Beispiel 5.

### Beispiel 7

Das Kopräzipitat für die Herstellung der aktiven Katalysatorphase wird erhalten, indem man 646,4 g $Fe(NO_3)_3 \cdot 9H_2O$, 174,6 g $Co(NO_3)_2 \cdot 6H_2O$, 1744,8 g $Ni(NO_3)_2 \cdot 6H_2O$ und 0, 1 g $KNO_3$ in 3, 1 l Wasser löst und unter Rühren bei 90° C zunächst eine Lösung von 34,9 g $Sm_2O_3$ in 212 g konz. $HNO_3$ zugibt. In dieser Lösung werden unter weiterem Rühren 600,9 g hochdisperse Kieselsäure (Aerosil 200) und 1201 ,8 g getemperter Montmorillonit (spez. Oberfläche nach BET < 1 m²/g) gegeben. In einem getrennten Gefäß wird bei 60° C eine Lösung von 2118,6 g $(NH_4)_6Mo_7O_{24} \cdot 6H_2O$ in 2,7 l $H_2O$ bereitet und zu dieser unter intensivem Rühren 115,3 g 85 %ige $H_3PO_4$ gegeben. Danach werden die beiden Lösungen unter intensivem Rühren vereinigt und eine Lösung von 727,7 g $Bi(NO_3)_3 \cdot 5H_2O$ in 612,0 g 8,2 %iger $HNO_3$ zugegeben. Die erhaltene Suspension wird in einem Sprühtrockner bei einer Eintrittstemperatur der Trocknungsluft von 550° C getrocknet. Das Verhältnis von Trocknungsluftmenge zu versprühter Suspensionsmenge wird dabei so eingestellt, daß eine Austrittstemperatur von 170° C erhalten wird und die Suspensionsmenge wird so eingestellt, daß eine Verweilzeit von 6 sec. des Sprühkorns im Sprühtrockner erhalten wird. Die Zerstäubungsintensität ist so gewählt, daß ein Sprühkorn mit einem mittleren Durchmesser $\overline{d}p$ = 25 μm erhalten wird. Das getrocknete Sprühkorn wird in einem Drehrohrofen bei einer Verweilzeit von 30 min. und einer maximalen Temperatur im Sprühkorn von 420° C kalziniert.

Die Weiterverarbeitung des kalzinierten Sprühkorns erfolgt wie in Beispiel 5.

Im Beispiel 8 wird die gleiche Katalysatorzusammensetzung wie in Beispiel 2 verformt, außer daß bei Beispiel 8 statt Pentaerythrit Cellulosepulver als Porenbildner eingesetzt wurde.

### Beispiel 8

Die Herstellung des kalzinierten Sprühkorns erfolgt wie in Beispiel 2.

1,6 kg des kalzinierten Sprühkorns werden in einer Kneter/Extruder-Kombination vorgelegt und mit 0,4 kg Cellulosepulver mit einem mittleren Korndurchmesser $\overline{d}p$ = 36 μm als Porenbildner 5 min. gemischt.

Die Weiterverarbeitung erfolgt wie in Beispiel 2.

Im Vergleichsbeispiel 3 wird der Katalysator wie in Beispiel 2 hergestellt, wobei aber bei der Verformung kein Porenbildner zugesetzt wird.

### Vergleichsbeispiel 3

Die Herstellung des kalzinierten Sprühkorns erfolgt wie in Beispiel 2.

1,6 kg des kalzinierten Sprühkorns werden in einer Kneter/Extruder-Kombination vorgelegt. Dazu werden 394,4 g einer 6 Gew.-%igen Tyloselösung gegeben, in der zuvor 21,3 g Petroleum emulgiert wurden. Diese Masse wird solange geknetet, bis ein homogener plastischer Zustand erreicht ist. Die Extrusion erfolgt in Form III von Figur 1 bei einem Druck von 14 bar und einer Temperatur von 32° C in der extrudierten Masse.

Die Weiterverarbeitung der extrudierten Masse erfolgt wie in Beispiel 2, wobei die Spitzentemperatur bei der Temperung im Drehrohr auf 620° C angehoben wird.

TABELLE 1    Katalysatorzusammensetzung (Atomzahlen)

| Beispiele | Vergl.-Beisp. | Mo | Fe | Co | Ni | Bi | P | K | Sm | Si (als Montmorillonit + hochdisp.Kiesels.) |
|---|---|---|---|---|---|---|---|---|---|---|
| 1 | 1 | 12 | - | - | - | 10,1 | 1,2 | - | - | 30 |
| 2,3,4,8 | 2,3 | 12 | 1,2 | 1,0 | 4,0 | 0,5 | 0,8 | 0,025 | 0,1 | 30 |
| 5 | | 12 | 0,6 | 0,8 | 6,0 | 1,0 | 1,0 | 0,05 | 0,2 | 10 |
| 6 | | 12 | 2,0 | 1,1 | 6,0 | 1,0 | 1,0 | 0,001 | 0,2 | 30 |
| 7 | | 12 | 1,6 | 0,6 | 6,0 | 1,5 | 1,0 | 0,001 | 0,2 | 30 |

EP 0 417 722 B1

**TABELLE 2**  physik. chem. Katalysatorcharakterisierung

| Beisp. | V-Beisp. | $O_p/V_{p-1}$ mm | $d_K^*$ mm | Porosität – | $V_{meso}$ ml/g | $V_{makro}$ ml/g | Schutt- g/ml | He- g/ml | Hg-Dichte g/ml | BET-Oberfl. $m^2/g$ | Bruchf. N | Abrieb mg/g | $\Delta p$ Pa/m |
|---|---|---|---|---|---|---|---|---|---|---|---|---|---|
| 1 | | 1,65 | 7,1 | 0,60 | 0,04 | 0,46 | 0,71 | 3,97 | 1,58 | 13 | 6,8 | 42 | 1440 |
| | 1 | 1,2 | 7,1 | 0,34 | 0,10 | 0,17 | 1,09 | 4,04 | 2,60 | 19 | 26,9 | 56 | 1620 |
| 2 | | 1,65 | 7,1 | 0,51 | 0,05 | 0,38 | 0,76 | 3,48 | 1,71 | 14 | 14,9 | 16 | 1450 |
| 3 | | 2,00 | 7,1 | 0,62 | 0,05 | 0,48 | 0,60 | 3,49 | 1,30 | 16 | 9,8 | 32 | 1400 |
| 4 | | 2,10 | 7,1 | 0,62 | 0,04 | 0,42 | 0,70 | 2,54 | 1,30 | 16 | 15,4 | 48 | 1340 |
| | 2 | 1,2 | 7,1 | 0,64 | 0,04 | 0,42 | 0,79 | 3,59 | 1,30 | 16 | 18,9 | 20 | 1520 |
| 5 | | 1,73 | 7,1 | 0,56 | 0,03 | 0,39 | 0,70 | 4,02 | 1,77 | 12 | 6,2 | 8 | 1260 |
| 6 | | 1,73 | 7,1 | 0,56 | 0,04 | 0,30 | 0,72 | 4,05 | 1,79 | 11 | 10,7 | 4 | 1190 |
| 7 | | 1,73 | 7,1 | 0,56 | 0,03 | 0,29 | 0,73 | 4,08 | 1,81 | 12 | 7,7 | 14 | 1290 |
| 8 | | 1,65 | 7,1 | 0,62 | 0,04 | 0,33 | 0,76 | 3,51 | 1,30 | 16 | 14,4 | 18 | 1430 |
| | 3 | 1,65 | 7,1 | 0,44 | 0,05 | 0,25 | 0,87 | 3,46 | 1,93 | 13 | 16,8 | 11 | 1400 |

$^*$ $d_K$ = Durchmesser der den Katalysator gerade noch umschließenden Kugel

EP 0 417 722 B1

EP 0 417 722 B1

**TABELLE 3**     **Katalysatorwirkung bei der Herstellung von Acrolein**

| Beisp. | V-Beisp. | Salzbadtemp. °C | Umsatz % | Exotherme grd | Acrol. Ausb. % | Acryls. Ausb. % | Acrol. Selekt. % | Acryls. Selekt. % | Produktivität g Acrol./dm³ Katal./h |
|--------|----------|-----------------|----------|---------------|----------------|-----------------|------------------|-------------------|--------------------------------------|
| 1 | | 431 | 84,0 | 36 | 56,1 | 5,7 | 66,8 | 6,8 | 221 |
| | 1 | 422 | 82,5 | 38 | 50,9 | 5,2 | 61,7 | 6,3 | 180 * |
| 2 | | 331 | 94,2 | 81 | 81,8 | 7,0 | 86,8 | 7,4 | 322 |
| 3 | | 347 | 93,4 | 78 | 81,4 | 7,3 | 87,1 | 7,8 | 320 |
| 4 | | 345 | 92,7 | 76 | 80,9 | 7,1 | 87,2 | 7,7 | 318 |
| | 2 | 330 | 93,1 | 78 | 77,6 | 8,5 | 83,3 | 9,1 | 273 * |
| 5 | | 364 | 91,1 | 72 | 80,3 | 8,7 | 88,1 | 9,5 | 316 |
| 6 | | 344 | 90,9 | 76 | 80,1 | 9,0 | 88,1 | 9,9 | 315 |
| 7 | | 359 | 91,3 | 72 | 80,3 | 8,8 | 87,9 | 9,6 | 316 |
| 8 | | 348 | 92,6 | 77 | 80,4 | 6,9 | 86,8 | 7,5 | 317 |
| | 3 | 350 | 90,0 | 74 | 77,1 | 6,0 | 85,6 | 6,7 | 272 * |

\* Einspeisung 5,2 Mol Propen/h

**Patentansprüche**

1. Katalysator für die katalytlsche Gasphasenoxidation von Olefinen zu ungesättigten Aldehyden, insbesondere von Propen zu Acrolein, enthaltend als aktive Masse zumindest die Elemente Molybdän, Wismut, Phosphor und Sauerstoff in Atomverhältnissen

$$Mo_{12}Bi_{0,2-10}P_{0,2-5}O_x$$

sowie siliciumhaltiges Trägermaterial, wobei aktive Masse und Trägermaterlal zu Katalysatorkörpern von beliebiger geometrischer Form verformt sind, deren spezifische Oberfläche nach BET mindestens 10 $m^2/g$ beträgt,
**dadurch gekennzeichnet,**
daß die Katalysatorkörper bei einer Quecksilber dichte von mindestens 1,25 $g/cm^3$ eine Porosität von mindestens 0,46 aufweisen, die sich aus mindestens 0,03 $cm^3/g$ Mesoporenvolumen (2 - 30 nm) und mindestens 0,3 $cm^3/g$ Makroporenvolumen (> 30 nm) bei abwesenden Mikroporen (< 2 nm) zusammensetzt, daß die Katalysatorkörper eine solche geometrische Form aufweisen, daß deren Verhältnis von äußerer Oberfläche $O_p$ zu Volumen $V_p$ oberhalb von 1,6 $mm^{-1}$ liegt und deren räumliche Ausdehnung, beschrieben durch den Durchmesser einer die Formkörper gerade noch umschließenden Kugel, kleiner als 7,5 mm ist und diese Katalysatorkörper bei einer Katalysatorschüttung in einem Rohr mit 2 cm Durchmesser einem Druckverlust von unter 1600 Pa/m verursachen und daß die Katalysatorkörper eine Bruchfestlgkeit von mindestens 6,0 N und einen Abrieb von unter 50 mg/g Katalysator aufweisen.

2. Katalysator nach Anspruch 1,
**gekennzeichnet durch**
eine Masse, enthaltend die Zusammensetzung

$$Mo_{12}Fe_{0,4-4,0}Co_{0,4-4,0}Ni_{1,0-9,0}Bi_{0,2-2,0}$$
$$P(As)_{0,2-2,0}K(Rb,Cs)_{0-0,1}Sm_{0,01-0,2}Si_{5-40}O_x,$$

wobei das Element Silicium als pyrogene oder hochdisperse gefällte Kieselsäure, Kieselsäuresol, feinteiliges Aluminiumsilikat, insbesondere in Form von Montmorillonit vorliegt.

3. Katalysator nach Anspruch 2,
**gekennzeichnet durch**
eine Masse, enthaltend die Zusammensetzung

$$Mo_{12}Fe_{0,6-2,0}Co_{0,6-2,0}Ni_{2,0-6,0}Bi_{0,5-1,5}$$
$$P(As)_{0,5-1,5}K(Rb,Cs)_{0,001-0,05}Sm_{0,02-0,1}Si_{10-30}O_x$$

wobei das Element Silicium in Form von pyrogenem $SiO_2$ und Montmorillonit im Gewichtsverhältnis 1 : 0,5 bis 1 : 4 vorliegt.

4. Katalysator nach Anspruch 2 oder 3,
**dadurch gekennzeichnet,**
daß der Montmorillonit eine durch Glühbehandlung herabgesetzte spezifische Oberfläche nach BET unterhalb von 2,0 $m^2/g$ hat.

5. Verfahren zur Herstellung des Katalysators nach den Ansprüchen 1 bis 4,
**dadurch gekennzeichnet,**
daß man

a) eine in an sich bekannter Weise durch Vereinigen von Salzlösungen der katalytisch aktiven Elemente (außer Si) gewonnenen Suspension des Kopräzipitats mit dem unlöslichen siliciumhaltigen Feststoff mischt und die dabei erhaltene Suspension unter Bedingungen sprühtrocknet, welche eine Anfangstemperatur der Trocknungsluft von 300° C - 600° C und eine Temperatur bei der Abscheidung des getrockneten Pulvers von 120° C - 220° C sowie eine ein Sprühkorn mit einem mittleren Korndurchmesser kleiner 30 μm erzeugende Zerstäubungsintensität vorsehen, wobei die Verweilzeit des Sprühkorns im Trockner 2 bis 25 sec. beträgt,

b) das trockene Sprühkorn in einem Ofen, vorzugsweise einem Drehrohrofen, bei Verweilzeiten von 5 bis 60 min und einer Spitzentemperatur im Sprühkorn von 320 bis 480°C kalziniert,

c) das kalzinierte Sprühkorn mit 5 bis 40 Gew.-%,bezogen auf die Menge des Sprühkorns, eines bei einer Temperatur unter 400° C vollständig zersetzlichen Porenbildners und mit einer eine extrusionsfähige Masse ergebenden Menge an Befeuchtungs-, Gleit- und Bindemittel, deren Anteil insgesamt nicht mehr als 40 Gew.-% der Sprühkornmenge ausmacht, bei einer Temperatur unterhalb 80° C in der gewünschten geometrischen Form bei einem Druck unterhalb 50 bar extrudiert und den extrudierten Strang durch Schneiden auf die Länge des gewünschten Körpers unterteilt,

d) die extrudierten Einzelkörper trocknet, dann in einem Ofen, vorzugsweise einem Drehrohrofen, die enthaltene zersetzbare Substanz vorsichtig ausbrennt und dann bei einer Verweilzeit von 5 bis 60 min und einer in der Schüttung der Einzelkörper gemessenen Spitzentemperatur von 450 bis 650° C im Luftstrom tempert.

6. Verfahren nach Anspruch 5,
**dadurch gekennzeichnet,**
daß man als Porenbildner festen Pentaerythrit mit einem mittleren Korndurchmesser kleiner 40 μm einsetzt.

7. Verfahren nach den Ansprüchen 5 oder 6,
**dadurch gekennzeichnet,**
daß man als Gleitmittel Petroleum oder Wasser und als Befeuchtungs- und Bindemittel bzw. Gleitmittel eine 1 - 10 Gew.-%ige wäßrige Methylcelluloselösung, vorzugsweise in Form einer Öl- in Wasser-Emulsion, oder als Bindemittel trockenes Methylcellulosepulver einsetzt.

8. Verfahren nach den Ansprüchen 5 bis 7,
**dadurch gekennzeichnet,**
daß man Ausbrennen und Tempern der extrudierten Einzelkörper in gesonderten Schritten vornimmt.

9. Verfahren nach den Ansprüchen 5 bis 8,
**dadurch gekennzeichnet,**
daß man die extrudierten Einzelkörper und den Luftstrom beim Ausbrennvorgang im Gleichstrom führt und das Ausbrennen bei höchstens 400° C vornimmt.

**Claims**

1. A catalyst for catalytic gas-phase oxidation of olefins to unsaturated aldehydes, more particularly propene to acrolein, containing as active mass at least the elements molybdenum, bismuth, phosphorus and oxygen in atomic ratios of

$$Mo_{12} Bi_{0.2- 10} P_{0.2- 5} O_x$$

and silicon-containing support material, the active mass and the support material being formed into catalyst elements of any geometric shape having a specific BET surface of at least 10 $m^2/g$, characterized in that, for a mercury density of at least 1.25 $g/cm^3$, the catalyst elements have a porosity of at least 0.46 made up of at least 0.03 $cm^3/g$ mesopore volume (2 to 30 nm) and at least 0.3 $cm^3/g$ macropore volume (> 30 nm) in the absence of micropores (< 2 nm), in that the catalyst elements have such a geometric shape that their ratio of outer surface Op to volume Vp is above 1.6 $mm^{-1}$ and their spatial dimension, defined by the diameter of a sphere which still just surrounds it, is smaller than 7.5 mm and in that, in a catalyst bed in a 2 cm diameter tube, the catalyst elements cause a pressure loss of less than 1600 Pa/m and in that the catalyst elements have a breaking strength of at least 6.0 N and an abrasion of less 50 mg/g catalyst.

2. A catalyst as claimed in claim 1, characterized by a mass having the composition:

$$Mo_{12}Fe_{0.4- 4.0}Co_{0.4- 4.0}Ni_{1.0- 9.0}Bi_{0.2- 2.0}$$
$$P(As)_{0.2- 2.0}K(Rb,Cs)_{0- 0.1}Sm_{0.01- 0.2}Si_{5-40}O_x,$$

the element silicon being present in the form of pyrogenic or highly disperse precipitated silica, silica sol, finely divided aluminium silicate and, more particularly, in the form of montmorillonite.

3. A catalyst as claimed in claim 2, characterized by a mass having the following composition:

$$Mo_{12}Fe_{0.6- 2.0}Co_{0.6- 2.0}Ni_{2.0- 6.0}Bi_{0.5- 1.5}$$
$$P(As)_{0.5- 1.5}K(Rb,cs)_{0.001- 0.05}Sm_{0.02- 0.1}Si_{10-30}O_x,$$

the element silicon in the form of pyrogenic $SiO_2$ and montmorillonite being present in a ration by weight of 1:0.5 to 1:4.

4. A catalyst as claimed in claim 2 or 3, characterized in that the montmorillonite has a specific BET surface reduced by calcination of less than 2.0 $m^2/g$.

5. A process for the production of the catalyst claimed in claims 1 to 4, characterized in that

a) a suspension of the co-precipitate obtained in known manner by combining salt solutions of the catalytically active elements (except Si) is mixed with the insoluble silicon-containing solid and the suspension obtained is spray-dried under conditions which provide for an initial temperature of the drying air of 300°C to 600°C, a temperature during deposition of the dried powder of 120°C to 220°C and a spraying intensity designed to produce a spray-dried powder having an average particle diameter of less than 30 μm, the residence time of the spray-dried powder in the dryer being from 2 to 25 seconds,

b) the spray-dried powder is calcined in a furnace, preferably a rotary kiln, over residence times of 5 to 60 minutes and at a peak temperature in the spray-dried powder of 320 to 480°C,

c) the calcined spray-dried powder is extruded with 5 to 40% by weight, based on the quantity of spray-dried powder, of a pore-forming agent which decomposes completely at a temperature below 400°C and with wetting agents, lubricants and binders in a quantity sufficient to produce an extrudable mass, the wetting agents, lubricants and binders together making up no more than 40% by weight of the quantity of spray-dried powder, in the required geometric shape at a temperature below 80°C and under a pressure below 50 bar and the extruded strand is divided up by cutting to the length of the desired element.

d) the extruded individual elements are dried, the decomposable substance present is carefully removed by burning out in a furnace, preferably a rotary kiln, and the individual elements are conditioned in an air stream for 5 to 60 minutes at a peak temperature, as measured in the bed of individual elements, of 450 to 650°C.

6. A process as claimed in claim 5, characterized in that solid pentaerythritol having an average particle diameter of less than 40 μm is used as the pore-forming agent.

7. A process as claimed in claim 5 or 6, characterized in that petroleum or water is used as the lubricant and a 1 to 10% by weight aqueous methyl cellulose solution, preferably in the form of an oil-in-water emulsion, is used as the wetting agent and binder or lubricant or dry methyl cellulose powder is used as the binder.

8. A process as claimed in claims 5 to 7, characterized in that burning out and conditioning of the extruded individual elements are carried out in separate steps.

9. A process as claimed in claims 5 to 8, characterized in that the extruded individual elements and the air stream are moved in co-current during the burning-out step and the burning-out step is carried out at a temperature of at most 400°C.

## Revendications

1. Catalyseur pour l'oxydation catalytique en phase gazeuse d'oléfines en aldéhydes insaturés, en particulier du propène en acroléine, contenant en tant que matière active au moins les éléments molybdène, bismuth, phosphore et oxygène en rapports pondéraux

$$MO_{12} Bi_{0,2-10} P_{0,2-5}O_X$$

ainsi qu'un matériau de support contenant du silicium, la matière et le matériau de support étant mis en forme pour donner des corps de catalyseur de forme géométrique quelconque, dont la surface spécifique selon BET est au moins de 10 m²/g,

caractérisé en ce que les corps de catalyseur, à une densité au mercure d'au moins 1,25 g/cm³, présentent une porosité d'au moins 0,46, qui se compose d'un volume de mésopores (2-30 nm) d'au moins 0,03 cm³/g et d'un volume de macropores (>30 nm) d'au moins 0,3 cm³/g avec une absence de micropores (<2 nm), en ce que les corps de catalyseur présentent une forme géométrique telle que leur rapport de la superficie externe $O_p$ au volume $V_p$ est supérieur à 1,6 mm$^{-1}$ et dont l'expansion spatiale, décrite par le diamètre d'une sphère comprenant juste les corps moulés, est inférieure à 7,5 mm, et ces corps de catalyseur, dans le cas d'un lit de catalyseur dans un tube de 2 cm de diamètre, provoquent une chute de pression de moins de 1600 Pa/m, et en ce que les corps de catalyseur présentent une résistance à la rupture d'au moins 6,0 N et une abrasion de moins de 50 mg/g de catalyseur.

2. Catalyseur selon la revendication 1, caractérisé par une matière ayant la composition :

$$Mo_{12}Fe_{0,4-4,0}CO_{0,4-4,0}Ni_{1,0-9,0}Bi_{0,2-2,0}$$
$$P(As)_{0,2-2,0}K(Rb,Cs)_{0-0,1}Sm_{0,01-0,2}Si_{5-40}O_X$$

l'élément silicium se trouvant sous forme d'acide silicique pyrogéné ou précipité fortement dispersé, de sol d'acide silicique, d'aluminosilicate finement divisé, en particulier sous forme de montmorillonite.

3. Catalyseur selon la revendication 2, caractérisé par une matière ayant la composition :

$$Mo_{12}Fe_{0,6-2,0}Co_{0,6-2,0}Ni_{2,0-6,0}Bi_{0,5-1,5}$$
$$P(As)_{0,5-1,5}K(Rb,Cs)_{0,001-0,05}Sm_{0,02-0,1}Si_{10-30}O_X,$$

l'élément silicium sous forme de $SiO_2$ pyrogéné et de montmorillonite se trouvant en rapport pondéral de 1:05 à 1:4.

4. Catalyseur selon la revendication 2 ou 3, caractérisé en ce que la montmorillonite a une surface spécifique BET, déterminée par traitement de cuisson, inférieure à 2,0 m$^2$/g.

5. Procédé pour la préparation du catalyseur selon l'une quelconque des revendications 1 à 4, caractérisé en ce que :

a) on mélange une suspension du co-précipité, obtenu d'une façon connue en soi par réunion de solutions de sels des éléments catalytiquement actifs (à l'exception de Si) avec le solide insoluble contenant du silicium, et on sèche par atomisation la suspension ainsi obtenue, dans des conditions qui prévoient une température initiale de l'air de séchage de 300 à 600°C et une température de 120-220°C lors de la séparation de la poudre séchée, ainsi qu'une intensité d'atomisation engendrant un grain atomisé ayant un diamètre de grain moyen inférieur à 30 μm, le temps de séjour du grain atomisé dans le sécheur allant de 2 à 25 secondes ;

b) on calcine le grain atomisé sec dans un four, de préférence un four tubulaire rotatif, avec des temps de séjour de 5 à 60 minutes et à une température maximale dans le grain atomisé de 320 à 480°C ;

c) on extrude à la forme géométrique désirée le grain atomisé calciné, avec de 5 à 40 % en poids, par rapport à la quantité de grain atomisé, d'un agent porogène totalement décomposable à une température inférieure à 400°C, et avec une quantité, donnant une masse apte à l'extrusion, de liant, agent mouillant et lubrifiant, dont la proportion au total ne constitue pas plus de 40 % en poids de la quantité des grains atomisés, à une température inférieure à 80°C, sous une pression inférieure à 50 bars, et on divise le boudin extrudé par tranchage à la longueur du corps recherché ;

d) on sèche les corps individuels extrudés, puis on élimine avec précaution la substance décomposable contenue, par cuisson dans un four, de préférence un four tubulaire rotatif, et on les soumet ensuite à un traitement thermique dans un courant d'air pendant un temps de séjour de 5 à 60 minutes et à une température maximale, mesurée dans le lit des corps individuels, de 450 à 650°C.

6. Procédé selon la revendication 5, caractérisé en ce que l'on utilise en tant qu'agent porogène du pentaérythritol solide ayant un diamètre moyen de particules inférieur à 40 μm.

7. Procédé selon la revendication 5 ou 6, caractérisé en ce que l'on utilise en tant que lubrifiant du pétrole ou de l'eau et, en tant que liant et agent mouillant ou lubrifiant, une solution aqueuse de méthylcellulose à 1-10 % en poids, de préférence sous forme d'une émulsion huile-dans-l'eau ou, en tant que liant, de la poudre de méthylcellulose sèche.

8. Procédé selon l'une quelconque des revendications 5 à 7, caractérisé en ce que l'on effectue en étapes séparées l'extraction par cuisson et le traitement thermique des corps individuels extrudés.

9. Procédé selon l'une quelconque des revendications 5 à 8, caractérisé en ce que dans l'opération d'extraction par cuisson, on dirige les corps individuels extrudés et le courant d'air en écoulement de même sens, et on effectue l'extraction par cuisson au maximum à 400°C.

*Fig. 1*

I

II

III

Fig. 2

IV

V

VI